# EUROPEAN PATENT APPLICATION

(11) **EP 4 190 825 A1**
(43) Date of publication of application: **07.06.2023**
(21) Application number: 21896079.7
(22) Date of filing: 24.02.2021
(51) Int. Cl.: C08F 212/08, C08F 257/02, C08J 9/00, B01J 20/285, B01J 20/26

(54) **SOLID-PHASE SYNTHESIS CARRIER, PREPARATION METHOD THEREFOR AND USE THEREOF**

(30) Priority: 26.11.2020 CN 202011357543
(71) Applicant: Sunresin New Materials Co. Ltd., Xi'an, Shaanxi 710075 (CN)
(72) Inventor: WANG, Chaoyang, Shaanxi 710075 (CN); ZHANG, Cheng, Shaanxi 710075 (CN); WU, Dan, Shaanxi 710075 (CN); ZHAO, Weijie, Shaanxi 710075 (CN); LI, Yanjun, Shaanxi 710075 (CN); LIU, Qiong, Shaanxi 710075 (CN); KOU, Xiaokang, Shaanxi 710075 (CN)
(74) Representative: Symbiosis IP Limited
(86) International application number: PCT/CN2021/077661
(87) International publication number: WO 2022/110559

(57) **Abstract**

A solid-phase synthesis carrier, a preparation method therefor and the use thereof, wherein a diene cross-linking agent with a similar reactivity ratio to styrene and two vinyl groups thereof not on the same benzene ring is selected as a cross-linking monomer, and is subjected to suspension polymerization to obtain a porous resin. The porous resin is then functionalized to obtain a porous resin with an amino or hydroxyl functional group. Compared with existing preparation methods, the reactivity ratio of the cross-linking agent and styrene is similar, which is beneficial to improving the uniformity of the chemical structure in the resin, forming uniformly distributed active sites and channels, and is beneficial to improving the reaction efficiency and reducing the mass transfer resistance. The preparation of oligonucleotides by using such a carrier as a solid-phase synthesis carrier can improve the yield and purity of the oligonucleotides.

## Description

### TECHNICAL FIELD

The invention relates to a solid-phase synthesis carrier, preparation method thereof and use thereof. The carrier is for use in the solid-phase synthesis of oligonucleotides and belongs to the field of preparation of functional polymer materials.

### BACKGROUND

As one of the effective tools for precision medicine, more and more oligonucleotide drugs are being approved for marketing. Oligonucleotides drugs are often synthesized using chemical methods, and the most common method is solid-phase phosphoramidite synthesis, in which a solid-phase synthesis carrier is filled in a reaction column and a solution with dissolved reactants is rapidly flowed through the column under a certain pressure to complete the reaction.

At the early stage of the development of oligonucleotide solid-phase synthesis technology, the commonly used solid-phase synthesis carriers include inorganic particles such as controlled pore glass microspheres (CPG) and modified silica, but they have obvious drawbacks such as low loading (generally less than 100 µmmol/g), which leads to the limited yield of single batch of oligonucleotide, low equipment utilization and high production cost. In order to improve the loading of the carrier, Nitto Denko CO. LTD. and Ionis Inc. jointly filed a patent application WO2006029023, which involved preparing organic polymers as carries for oligonucleotides solid-phase synthesis, using styrene, acetoxystyrene and divinylbenzene as polymerization monomers and isooctane and 2-ethyl-1-hexanol as pore-forming agents. The prepared carrier had a loading of up to 100-350 µmmol/g. In order to further improve the yield and purity of oligonucleotides, patent application US8653152 filed by Nitto Denko CO. LTD use styrene, methacrylonitrile, acetoxystyrene and divinylbenzene as polymerization monomers, isooctane, 2-ethyl-1-hexanol as pore-forming agent to prepare a solid-phase synthesis carrier. The synthesis efficiency is improved by adding methacrylonitrile to suppress the swelling fluctuation of the solid-phase synthesis carrier in different solvents.

Both of the above polymer carriers use divinylbenzene as a cross-linking agent. During use, divinylbenzene has two main problems, one is that the purity is not high, and the other is that it has three isomers with different properties. This leads to a great difference in the performance of the resins produced using different quality divinylbenzene from different manufacturers. At present, the purity of general industrial divinylbenzene is 40-60%. In addition to divinylbenzene, it also contains a certain amount of ethyl styrene, and some also contain a small amount of diethylbenzene.

The three isomers of divinylbenzene show different reactivity when copolymerizing with other monomers, which has a significant impact on the structure and performance of the copolymer. In a study on the copolymerization kinetics of styrene-divinylbenzene by He Binglin et al. (Reactive Polymer, 12 (1990), 269-275), it was found that p-divinylbenzene and m-divinylbenzene have faster conversion rate than styrene in the copolymerization process. This leads to high cross-linking degree of copolymers formed in the early stage of polymerization and low cross-linking degree of copolymers formed in the late stage of polymerization, thus leading to heterogeneous chemical structure in the resin. Due to the heterogeneous chemical structure, the macroporous adsorption resin with styrene-divinylbenzene as the skeleton has a series of problems during use, such as varying degrees of fragmentation, long time to reach the adsorption equilibrium, tailing after desorption, incomplete regeneration, etc.

Diene cross-linking agents such as 1, 2-bis (4-vinylphenyl) ethane, where two vinyl groups are not on the same benzene ring, have a reactivity similar to styrene. Sundell et al. (Journal of Polymer Science Part A: Polymer Chemistry, 31 (1993), 2305-2311) prepared strong acid resins using this kind of monomers as cross-linking agents. Experimental results showed that these resins had higher mechanical strength than strong acid resins based on styrene-divinylbenzene copolymers. In a Research on Synthesis and Adsorption Performance of 1,2-bis (p-vinylphenyl) ethane Polymers, ultrahigh crosslinked polystyrene adsorption resins were prepared with 1,2-bis (p-vinylphenyl) ethane and divinylbenzene as cross-linking agents respectively. Experimental results showed that the adsorption resins with 1,2-bis (p-vinylphenyl) ethane as a cross-linking agent had no tailing phenomenon during use and were easy to regenerate.

Oligonucleotide drugs have emerged in the treatment of diseases such as anti-virus, high cholesterol, gene expression editing, visual blindness and hepatic vein obstruction due to their high therapeutic efficiency, low drug toxicity, strong specificity and wide application fields. The demand for oligonucleotide drugs is increasing year by year. At the same time, the existing carriers for oligonucleotide solid-phase synthesis have problems such as heterogeneous internal structure and high mass transfer resistance, which lead to low oligonucleotide synthesis efficiency and high production cost. Therefore, it is necessary to develop a solid-phase synthesis carrier which allows large scale synthesis of oligonucleotide drugs at low cost and with high efficiency to meet the market demand of oligonucleotide drugs.

### SUMMARY OF THE INVENTION

In order to achieve the large-scale production of oligonucleotides at low-cost and overcome the disadvantages such as heterogeneous internal structure and high mass transfer resistance of existing carriers for solid-phase synthesis of oligonucleotides, the present invention provides a solid-phase synthesis carrier and preparation method thereof and use thereof.

The present invention selects diene compounds, which have two vinyl groups that are not on the same benzene ring, as cross-linking monomers, showing mainly the following three effects: Firstly, such type of monomers have a reactivity ratio similar to styrene, which is conducive to improving the uniformity of the chemical structure inside the resin and forming uniformly distributed active sites. Secondly, the improvement in homogeneity of the chemical structure inside the resin is beneficial to formation of uniform pore channels inside the resin, which reduces the mass transfer resistance. Thirdly, compared with divinylbenzene, such type of cross-linking agents contain flexible molecular chains which can improve the swelling performance of the resin in different organic solvents.

The present invention provides a solid-phase synthesis carrier, wherein the carrier has a polymer skeleton with functional groups which is represented by the following formula: 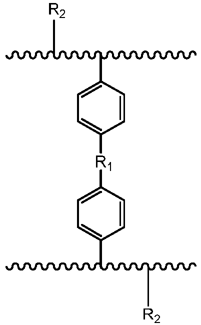 wherein, R₁= -(CH₂)ₙ-, n is an integer of 0-3, or R₁= -O-(CH₂)ₘ-O-, m is an integer of 1-4; and R₂= -OH or -NH₂.

In some embodiments, the solid-phase synthesis carrier is a copolymer comprising repeating structural units represented by formula (I), formula (II), formula (III), and formula (IV) in its skeleton: 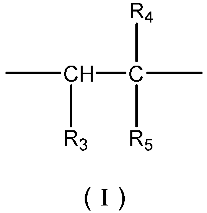
wherein, R₃ is selected from the groups consisting of -H, -CN and -CH₂-CN; R₄ is -H or -CH₃; and R₅ is selected from the groups consisting of -CN, -CH₂-CN, -OOCH₃ and -CONH₂; 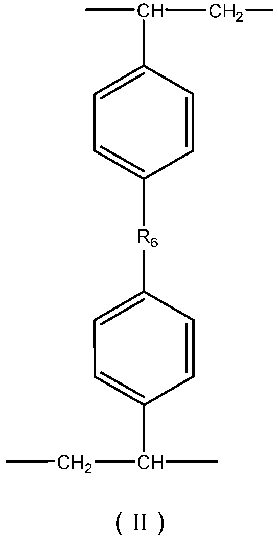
wherein, R₆ is -(CH₂₎ₓ-, x is an integer 0-3, or R6 is -O-(CH₂)_{y}-O -, y is an integer 1-4; 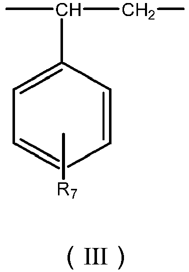
R₇ is selected from the groups consisting of -H, CH₃(CH₂)_{z} - or CH₃(CH₂)_{Z}O-, wherein z is an integer 0-4, (CH₃)₂CH-, (CH₃)₂CH(CH₂)-, (CH₃)₂CH(CH₂)₂-, (CH₃)₃C-, CH₃CH₂CH(CH₃)-, CH₃CH₂C(CH₃)₂-, and CH₃CH₂CH₂CH(CH₃)-; 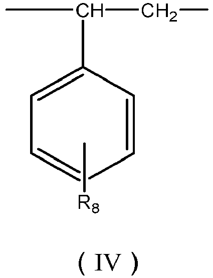
R₈ is selected from the groups consisting of -OH, -CH₂OH, -NH₂, -CH₂NH₂, -CHCOOC-C₆H₄-OH, -CHCOOCCH₂-C₆H₄-OH, -(CH₂)₄OOC-C₆H₄-OH, -(CH₂)₄OOCCH₂-C₆H₄-OH, -(CH₂)₄OOCCH₂-C₆H₄-NH₂, -CH₂COONH-C₆H₄-NH₂, -COO-C₆H₄-OH and -CH₂COO-C₆H₄-OH.

In some embodiments, the carrier has a content of hydroxyl group or amino group of 100-1000 µmmol/g, preferably 400-700 µmmol/g.

In some embodiments, the carrier has a particle size in a range of 35-200 µm, preferably 50-100 µm.

In some embodiments, the carrier has an average pore diameter of 10-200nm, preferably 40-100nm.

The present invention also provides a preparation method of the solid-phase synthesis. The preparation method comprises following steps of: preparing an aqueous phase and an oil phase, respectively; the aqueous phase comprising water, a dispersant and an inorganic salt; the oil phase comprising: a cross-linking monomer, a monovinyl compound, a functional monomer, a modified monomer, a pore-forming agent and an initiator, wherein the cross-linking monomer, the monovinyl compound, the functional monomer and the modified monomer are monomers capable of polymerization; and adding the oil phase to the aqueous phase, stirring and heating to carry out reaction, and removing the pore-forming agent after the reaction is completed, obtaining a porous polymer resin. The porous polymer resin is capable of undergoing a further reaction to obtain a solid-phase synthesis carrier containing a hydroxyl group or an amino group as functional groups.

In some embodiments, more specifically, the cross-linking monomer is a diene cross-linking agent which has two vinyl groups not on the same benzene ring, selected from the group consisting of 4,4'-divinylbiphenyl, di(4-vinylphenyl) methane, 1,2-di(4-vinylphenyl) ethane, 1,3-di(4-vinylphenyl) propane, di(4'-vinylphenoxy) methane, 1,2-di(4'-vinylphenoxy) ethane, 1,3-di(4'-vinylphenoxy) propane, 1,4-di(4'-vinylphenoxy) butane and any combination thereof. Preferably, the cross-linking monomer is 1,2-di(4-vinylphenyl) ethane

In some embodiments, the monovinyl compound is an aromatic monovinyl compound. The monovinyl compound is styrene, unsubstituted or substituted with C1-C5 alkyl on its benzene ring, such as methyl styrene, ethyl styrene, n-propyl styrene, isopropyl styrene, n-butyl styrene, isobutyl styrene, sec butyl styrene, tert butyl styrene, n-amyl styrene, isopentyl styrene, sec amyl styrene or tert amyl styrene; or styrene substituted with C1-C5 alkoxy, such as methoxy styrene, ethoxy styrene, propoxy styrene, butoxy styrene or pentoxy styrene. Preferably, the monovinyl compound is styrene.

In some embodiments, the functional monomer has a double bond capable of free radical polymerization, and also has a hydroxyl group, an amino group, a halogenated group or other group capable of converting into a hydroxyl group and an amino group via reaction. In the process of oligonucleotide synthesis, the reactive hydroxyl group or amino group works as active sites to connect oligonucleotides, including amino group, aminoalkyl group, hydroxyl group and hydroxyalkyl group etc. Primary amino group, aminomethyl group, hydroxyl group and hydroxymethyl group, etc. are preferred. The functional monomer comprises but is not limited to hydroxystyrene and derivatives thereof, such as 4-hydroxystyrene etc.; hydroxyalkyl styrene and derivatives thereof, such as 4-hydroxymethyl styrene etc.; acyloxy styrene and derivatives thereof, such as 4-acetoxy styrene and benzoyloxy styrene etc.; amino styrene and derivatives thereof, such as 4-amino styrene etc.; aminoalkyl styrene and derivatives thereof, such as 4-aminomethyl styrene etc.; haloalkyl styrene monomers, such as 4-(4-bromobutyl) styrene and 4-chloromethyl styrene etc.; 4-vinylphenyl ester monomers, such as methyl 4-vinylbenzoate and 4-ethenylbenzeneacetic acid ethyl ester ect..

In some embodiments, some of the functional monomers contain hydroxyl protection groups or amino protection groups which can be directly cut off to form amino groups or hydroxyl groups. For example, acyloxy styrene can be converted via alkali hydrolysis or acid hydrolysis into hydroxyl groups for serving as the active sites for connecting oligonucleotides; Some of the functional monomers need to be converted through a functionalization reaction into amino groups or hydroxyl groups for serving as active sites. For example, haloalkyl styrene can be converted through hydrolysis into hydroxyl group or converted through Gabriel reaction into primary amino groups for serving as active sites for connecting oligonucleotides; Some of the functional monomers need to link connecting arms having amino or hydroxyl groups for serving as active sites. For example, haloalkyl styrene can react with 4-hydroxybenzoic acid or 4-hydroxyphenylacetic acid to generate hydroxyl groups for serving as active sites for connecting oligonucleotides; Some of the functional monomers need the above multiple reactions to obtain amino groups or hydroxyl groups for serving as active sites. For example, 4-vinylphenyl esters monomers need to hydrolyze to expose carboxyl groups first, and then react with hydroquinone or p-phenylenediamine to obtain amino groups or hydroxyl groups for serving as active sites.

In some embodiments, the modified monomer has a double bond capable of free radical polymerization and also has cyano group, ester group and amide group. The modified monomer comprises but is not limited to acrylonitrile, methacrylonitrile, fumaronitrile, 1,4-dicyano-2-butene, methyl methacrylate and acrylamide.

In some embodiments, the initiator is selected from the group consisting of organic peroxides and azo compounds. The initiator comprises but is not limited to benzoyl peroxide, lauroyl peroxide, tert butyl peroxy-2-ethylhexanoate, 2,2'-azobis(2-methylpropionitrile), 2,2'-azobis(2-methylbutyronitrile) and 2,2'-azobis(2,4-dimethyl)valeronitrile. The initiator accounts for 0.5-5% by weight based on a total weight of the monomers.

In some embodiments, the pore-forming agent is an organic solvent or a surfactant which is not polymerizable and insoluble or slightly soluble in water. The pore-forming agent is selected from the group consisting of aromatic hydrocarbon such as benzene, toluene and ethylbenzene; aliphatic hydrocarbons, such as C6-C12 linear or branched alkanes or C6-C12 cycloalkanes, such as hexane, heptane, octane, dodecane, isooctane, isododecane and cyclohexane; halogenated hydrocarbons such as chloroform and chlorobenzene; esters containing 4 or more carbon atoms, such as ethyl acetate, butyl acetate and dibutyl phthalate; alcohols, such as C4-C12 linear or branched alkane alcohol or C4-C12 cycloalkane alcohol, such as hexanol, cyclohexanol, octanol, isooctanol, decanol and dodecanol; oil-soluble surfactants such as sorbitan trioleate, polyoxyethylene sorbitol beeswax derivative, sorbitan tristearate, polyoxyethylene sorbitol hexastearate, ethylene glycol fatty acid ester, propylene glycol fatty acid ester, propylene glycol monostearate, sorbitan sesquioleate, polyoxyethylene sorbitol oleate, monostearin, lanolin hydroxylated, sorbitol monooleate and propylene glycol laurate, and any combination thereof.

In some embodiments, the aqueous phase comprises water, a dispersant and an inorganic salt. The dispersant is a water-soluble polymer which comprises but is not limited to one or more selected from the group consisting of polyvinyl alcohol, hydroxyethyl cellulose, hydroxyethyl methyl cellulose, carboxymethyl cellulose, methyl cellulose, ethyl cellulose, sodium polyacrylate and polyvinylpyrrolidone. The dispersant is present in the aqueous phase in an amount of 0.1-5% by weight. The inorganic salt acts to regulate the density of the aqueous phase while reducing the solubility of the components of the oil phase in the aqueous phase, so that oil droplets are more stably dispersed in the aqueous phase. The inorganic salt comprises but is not limited to one or more selected from the group consisting of sodium chloride, potassium chloride, calcium chloride, sodium sulfate, potassium sulfate and calcium sulfate, etc. The inorganic salt is present in the aqueous phase in an amount of 20% by weight or lower.

In some embodiments, in order to reduce the bonding between resins and improve the heat transfer of polymerization, as well as to improve equipment utilization and production efficiency, a weight ratio of the oil phase to the aqueous phase is set to 1:3-1:20.

In some embodiments of the present invention, the different components are present in the following amounts: the monovinyl compound originally comprised in the oil phase accounts for 45-95% by weight based on a total weight of the monomers; the cross-linking monomer originally comprised in the oil phase accounts for 2.9-20% by weight based on a total weight of the monomers; the functional monomers originally comprised in the oil phase accounts for 2-20% by weight based on a total weight of the monomers; the modified monomer originally comprised in the oil phase accounts for 0.1-15% by weight based on a total weight of the monomers; and the pore-forming agent originally comprised in the oil phase accounts for 15-130% by weight based on a total weight of the monomers.

In some more preferred embodiments of the present invention, the different components are present in the following amounts: the monovinyl compound originally comprised in the oil phase accounts for 62-86% by weight based on a total weight of the monomers; the cross-linking monomer originally comprised in the oil phase accounts for 7-13% by weight based on a total weight of the monomers; the functional monomers originally comprised in the oil phase accounts for 5-15% by weight based on a total weight of the monomers; the modified monomer originally comprised in the oil phase accounts for 2-10% by weight based on a total weight of the monomers; and the pore-forming agent originally comprised in the oil phase accounts for 30-110% by weight based on a total weight of the monomers.

In some embodiments, the polymerization is carried out at a temperature of 50-90 °C, preferably, 70-85 °C.

In some embodiments, the preparation method of the solid-phase synthesis carrier comprises following steps of:
adding a certain amount of purified water into a reactor, adding the dispersant in an amount which is 0.1-5% by weight of the aqueous phase and the inorganic salt in an amount which is not more than 20% by weight of the aqueous phase, and dissolving to obtain an aqueous phase;
weighing out the monovinyl compound, cross-linking monomer, functional monomer, modified monomer, pore-forming agent and initiator according to a weight ratio of the oil phase to the aqueous phase being1:3-1:20;, wherein, the monovinyl compound accounts for 45-95% of the total weight of the monomers, the cross-linking monomer accounts for 2.9-20% of the total weight of the monomers, the functional monomer accounts for 2-20% of the total weight of the monomers, and the modified monomer accounts for 0.1-15% of the total weight of the monomers, the pore-forming agent accounts for 15-130% of the total weight of monomers, and mixing well to obtain an oil phase;
adding the oil phase into the reactor, stirring and heating up to 50-90 °C to carry out reaction; removing the pore-forming agent after the reaction is completed, and screening and collecting the resin with appropriate particle size, and vacuum drying to obtain a porous polymer resin; and carrying out a further reaction with the resin to obtain a solid-phase synthesis carrier having amino
group or carboxyl group.

In some embodiments, the preparation method of the solid-phase synthesis carrier comprises following steps of:
adding a certain amount of purified water into a reactor, adding the dispersant in an amount which is 0.1-5% by weight of the aqueous phase and the inorganic salt in an amount which is not more than 20% by weight of the aqueous phase, and dissolving to obtain an aqueous phase;
weighing out the monovinyl compound, cross-linking monomer, functional monomer, modified monomer, pore-forming agent and initiator according to a weight ratio of the oil phase to the aqueous phase being 1:3-1:20;, wherein, the monovinyl compound accounts for 62-86% of the total weight of the monomers, the cross-linking monomer accounts for 7-13% of the total weight of the monomers, the functional monomer accounts for 5-15% of the total weight of the monomers, and the modified monomer accounts for 2-10% of the total weight of the monomers, the pore-forming agent accounts for 30-110% of the total weight of monomers, and mixing well to obtain an oil phase;
adding the oil phase into the reactor, stirring and heating up to 70-85 °C to carry out reaction; removing the pore-forming agent after the reaction is completed, and screening and collecting the resin with appropriate particle size, and vacuum drying to obtain a porous polymer resin; and
carrying out a further reaction with the resin to obtain a solid-phase synthesis carrier having amino group or carboxyl group.

The above method enables to obtain the solid-phase synthesis of the present invention, i.e., a porous resin having hydroxyl groups or amino groups. In the present invention, the content of amino group or hydroxyl group, i.e., the loading, can be calculated by reacting with Fmoc-Leu-OH and then removing the Fmoc protecting group. The amount of Fmoc removed is determined by colorimetric method, and then the content of amino group or hydroxyl group in the carrier is calculated.

In some embodiments, the detailed operation is as follows: 1.0g of the carrier is accurately weighed and suspended in 7ml of acetonitrile solution, then 0.5g of Fmoc-Leu-OH, 0.5g of HBTU and 0.5ml of DIEA are added thereto, and stirred at room temperature to carry out reaction for 2h. After the reaction is completed, the resin is washed successively with acetonitrile (10ml for each time, three times) and methanol (10ml for each time, three times), and then the resin is dried. 0.1000g of the resin is weighed accurately, suspended in 20% piperidine/DMF (v/v) solution, and shaken for 30min at room temperature, filtered, and the filtrate is collected. The resin is washed with DMF and the filtrate is collected. The filtrates are combined to provide a total volume, and then diluted appropriate times to measure the absorbance at 300 nm. Similar Fmoc removal reactions are carried out by using a series of known concentrations of Fmoc-Leu-OH and the absorbance are measured to make a standard curve. The content of amino group or hydroxyl group in the carrier is calculated via the standard curve.

The functional group content of the carrier depends on the percentages of the functional monomers based on a total weight of the monomers, and a series of carriers with different amino group or hydroxyl group contents can be obtained by adjusting the amount of functional monomers. The functional group content of the carrier determines the amount of synthesized oligonucleotides; If the functional group content is too low, the yield of oligonucleotides in a single batch will be reduced; and if the functional group content is too high, the purity of oligonucleotides will be affected. In the present invention, the functional group content of the carrier ranges from 100-1000 µmmol/g, preferably 400-700 µmmol/g.

The particle size of the carrier in present invention is measured by particle image processing instrument. The carrier is uniformly distributed on a slide, and observed under a microscope at magnification, while images of the carrier particles under magnification are captured by a camera. Morphological features and particle size of the carrier are analyzed and calculated by a computer.

The particle size of the carrier mainly depends on the type and amount of dispersant present in the aqueous phase, the type and amount of pore-forming agents and the speed of stirring during the suspension polymerization. The particle size of the carrier can be regulated by adjusting above conditions. If the particle size of the carrier is too large, on the one hand, it will lead to the decrease of the specific surface area of the carrier and the increase of the number of active sites per unit area, which will affect the purity of oligonucleotide; on the other hand, if the particle size is too large, it will slow down the mass transfer rate and lead to the increase of impurities during the synthesis of oligonucleotides. If the particle size of the carrier is too small, it will cause too high pressure in the synthesis process and greatly increase the cost of equipment. In the present invention, the carrier has a particle size in a range of 35-200 µm, preferably 50-100 µm.

The average pore diameter of the carrier is measured by mercury intrusion method. 0.1500-0.3000g samples are precisely weighed and put into an automatic mercury porosimeter AutoPore IV 9500 (Micromeritics Instrument Co.). The contact angle of mercury is set to 130° and surface tension is set to 485dyn/cm, and then measured by mercury intrusion method under these conditions. The average pore diameter of the carrier mainly depends on the type and amount of pore-forming agents, the amount of cross-linking agent, the reaction temperature and time, etc. The average pore diameter of the carrier can be regulated by adjusting these conditions. If the average pore diameter of the carrier is too small, it will cause difficulties in mass transfer and affect the synthesis efficiency; if the average pore diameter of the carrier is too large, it will lead to the decrease of the specific surface area of the carrier and the increase of the active sites per unit area. During oligonucleotide synthesis, nucleoside growth will cause mutual influence and affect oligonucleotide purity. In the present invention, the carrier has an average pore diameter of 10-200nm, preferably 40-100 µm.

Compared with prior art, the present invention has three main advantages: Firstly, the present invention selects monomers which have a reactivity ratio similar to styrene as cross-linking agents, which is conducive to improving the uniformity of the internal chemical structure of the resin and forming uniformly distributed active sites. Secondly, the improvement in homogeneity of the internal chemical structure of the resin is conducive to the formation of uniform pore channels in the resin, thereby reducing the mass transfer resistance. Thirdly, compared with divinylbenzene, this kind of cross-linking agents contain flexible chains in its structure, which can improve the swelling performance of the resin in different organic solvents.

### BRIEF DESCRIPTION OF THE DRAWINGS

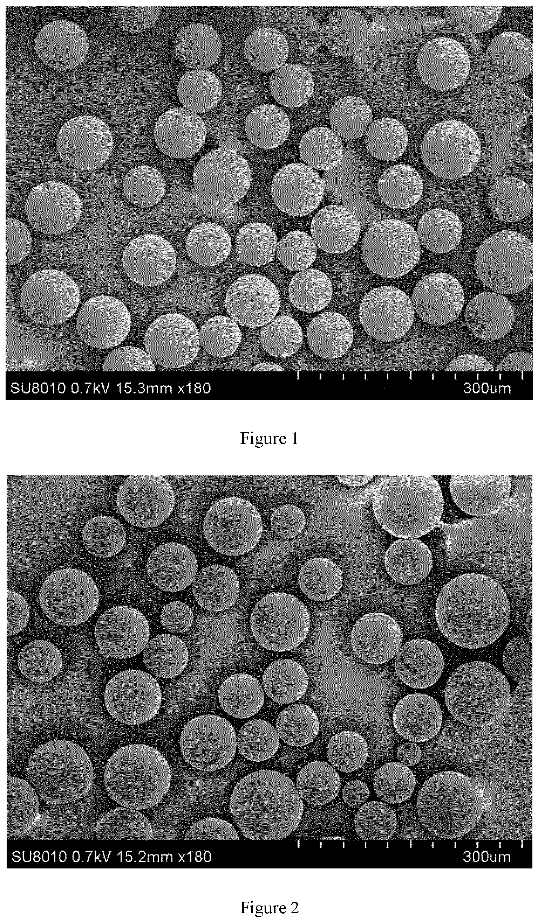

Figure 1 shows a scanning electron microscope image of the carrier in Example 1;
Figure 2 shows a scanning electron microscope image of the carrier in Example 2;

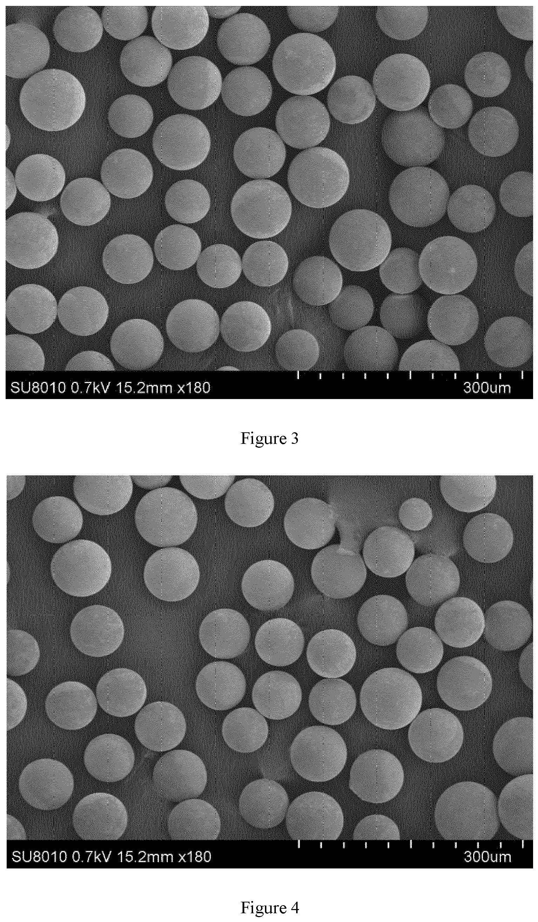

Figure 3 shows a scanning electron microscope image of the carrier in Example 3;
Figure 4 shows a scanning electron microscope image of the carrier in Example 4;

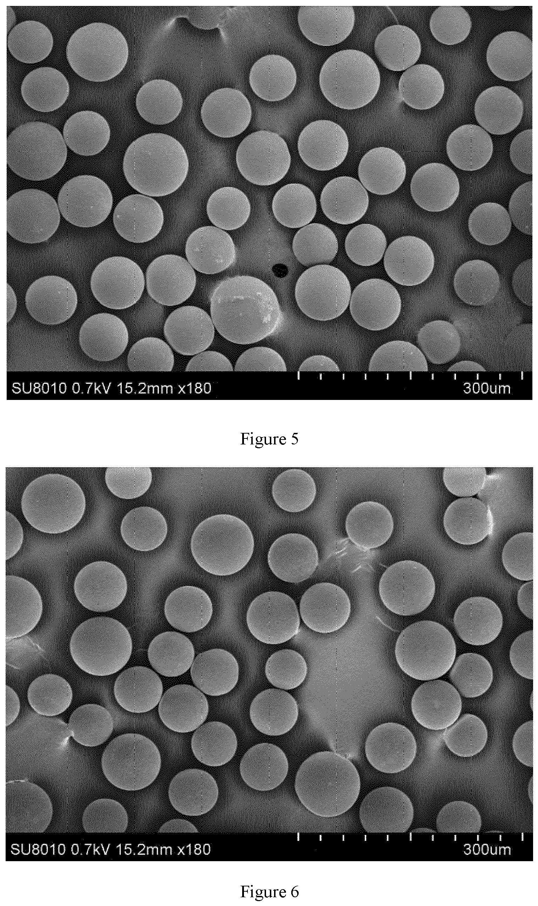

Figure 5 shows a scanning electron microscope image of the carrier in Example 5;
Figure 6 shows a scanning electron microscope image of the carrier in Example 6;

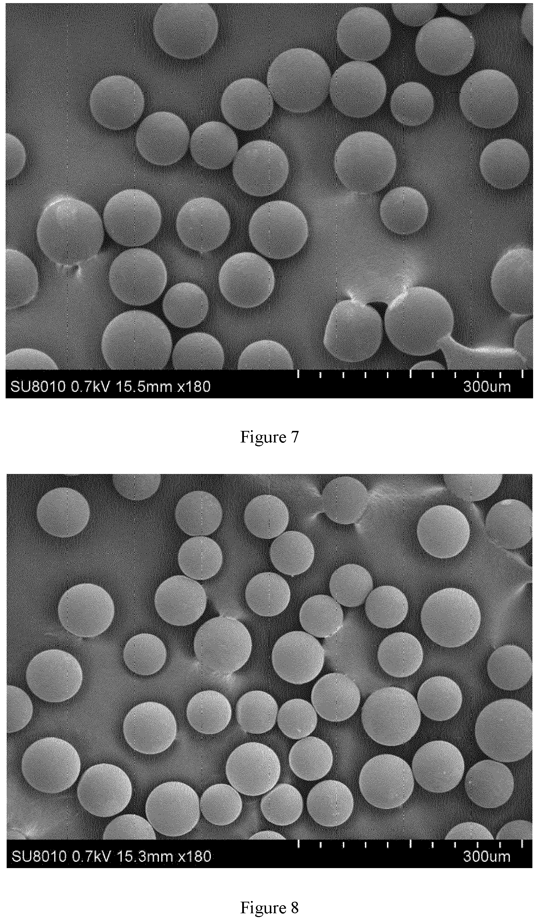

Figure 7 shows a scanning electron microscope image of the carrier in Example 7;
Figure 8 shows a scanning electron microscope image of the carrier in Example 8;

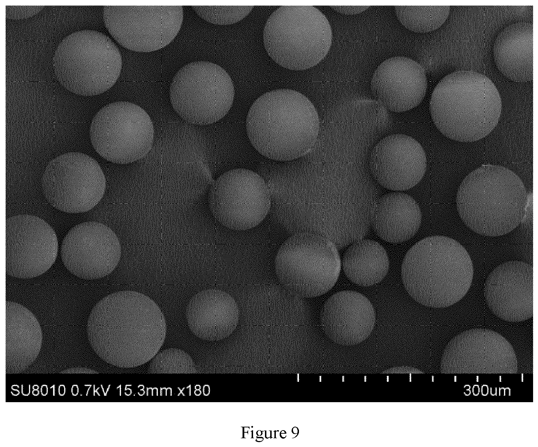

Figure 9 shows a scanning electron microscope image of the carrier in Example 9;

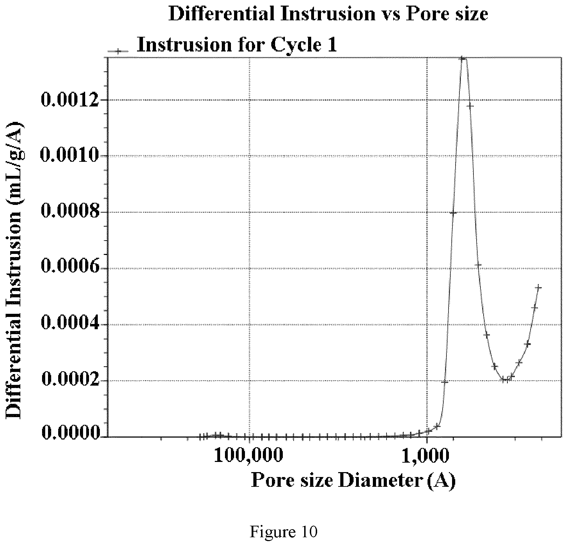

Figure 10 shows the pore size distribution of the carrier in Example 1 measured by mercury intrusion method;

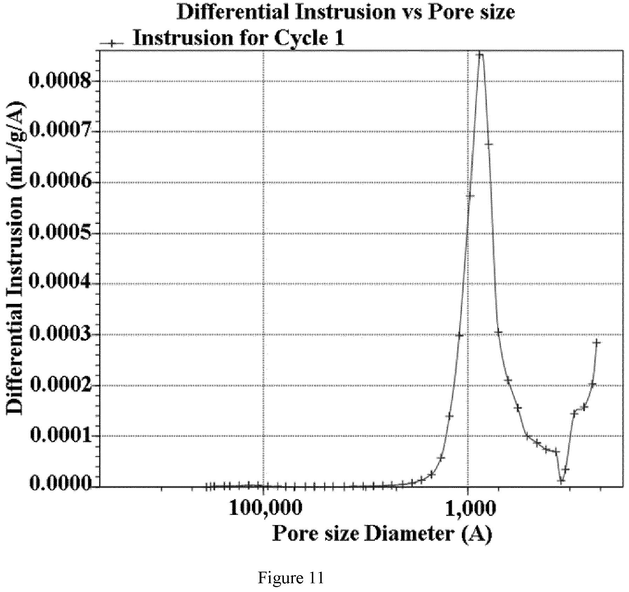

Figure 11 shows the pore size distribution of the carrier in Example 2 measured by mercury intrusion method;

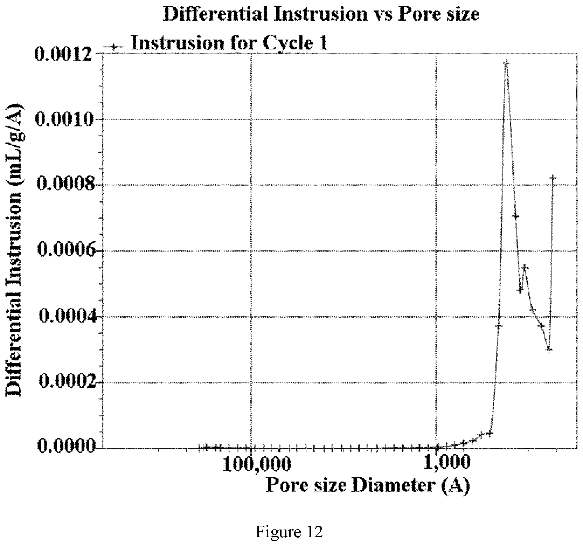

Figure 12 shows the pore size distribution of the carrier in Example 3 measured by mercury intrusion method;

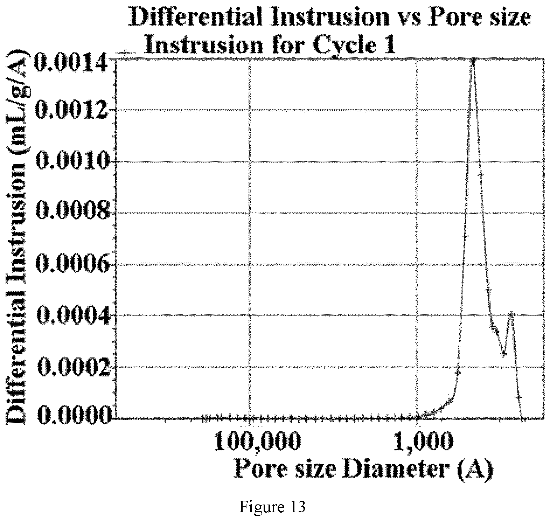

Figure 13 shows the pore size distribution of the carrier in Example 4 measured by mercury intrusion method;

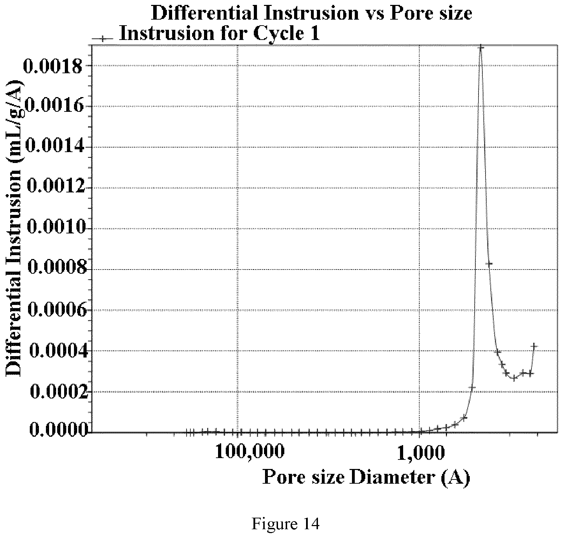

Figure 14 shows the pore size distribution of the carrier in Example 5 measured by mercury intrusion method;

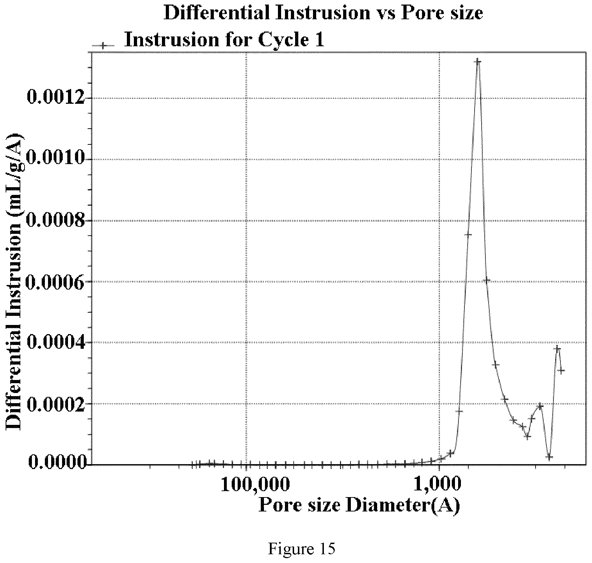

Figure 15 shows the pore size distribution of the carrier in Example 6 measured by mercury intrusion method;

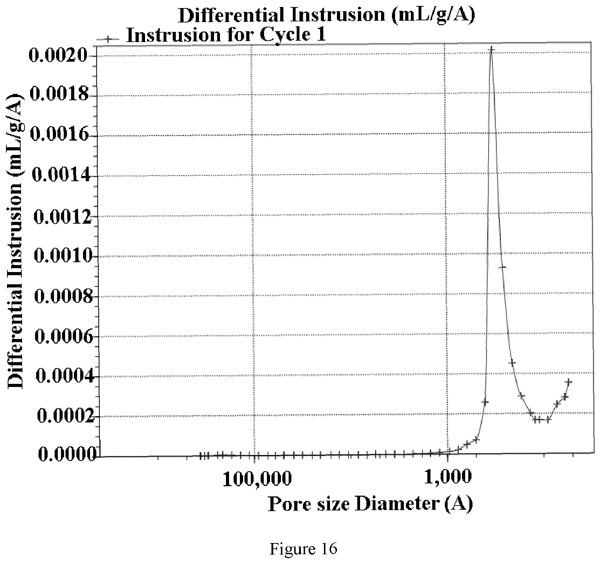

Figure 16 shows the pore size distribution of the carrier in Example 7 measured by mercury intrusion method;

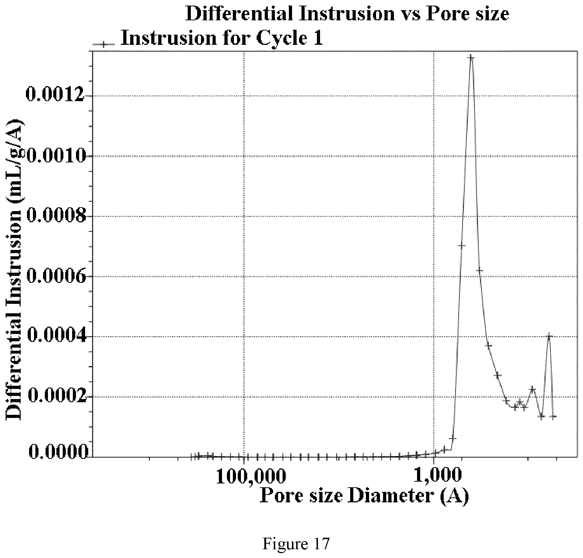

Figure 17 shows the pore size distribution of the carrier in Example 8 measured by mercury intrusion method;

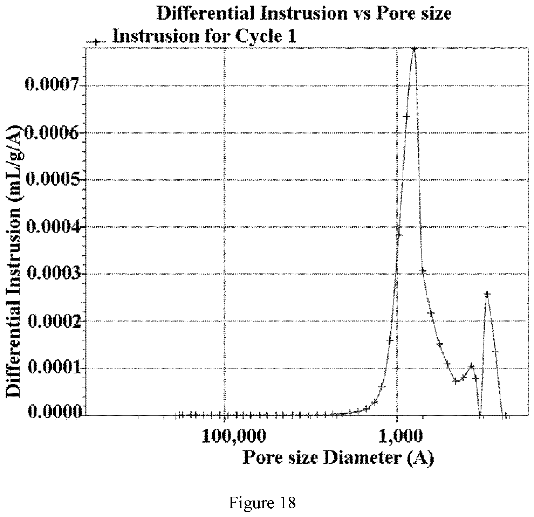

Figure 18 shows the pore size distribution of the carrier in Example 9 measured by mercury intrusion method.

### DETAILED DESCRIPTION OF THE EMBODIMENTS

The present application will be further illustrated with reference to following embodiments. However, these embodiments are only for illustrating, rather than limitations to the present invention detailed in the claims.

### Example 1

2L of purified water, 20g of polyvinyl alcohol and 30g of sodium chloride are added to a 3L reactor equipped with a condenser, an agitator and a thermometer, and dissolved to obtain an aqueous phase. 108.8g of styrene, 14g of 1,2-di (p-vinylphenyl) ethane, 12.2g of p-chloromethyl styrene, 5g of fumaronitrile, 6g of sorbitol trioleate, 40g of isooctanol, 20g of isododecane and 2.5g of benzoyl peroxide are weighed out and mixed well to obtain an oil phase. The oil phase is added to the reactor, stirred and heated to 80 °C to carry out polymerization for 6h. Wash with hot water after the polymerization is completed. The pore-forming agents are removed by ethanol reflux extraction. Resin with a particle size of 50-100 µm is screened out and collected, and thereafter vacuum dried to obtain a polymer porous resin with a chlorine content of 565 µmmol/g.

50g of the polymer porous resin and 500ml of N,N-dimethylformamide are added to a 1L reactor equipped with a condenser, an agitator and a thermometer, and stirred. Then 30g potassium phthalimide is added and the temperature is raised to 95 °C to carry out reaction for 16 hours. Cool to room temperature after reaction is completed, wash twice with N,N-dimethylformamide, wash to neutral with purified water, and wash three times with absolute ethanol, and then filter and dry the resin. 200g absolute ethanol and 50g hydrazine hydrate are added to the reactor and heated to a temperature of 75 °C to carry out reaction for 16 hours. Thereafter wash three times with ethanol/purified water solution with a volume ratio of 50:50, wash with purified water to neutral, wash three times with absolute ethanol, and filter and dry the resin. 200g absolute ethanol and 50g concentrated hydrochloric acid are added to the reactor and heated to a temperature of 60 °C to carry out reaction for 6h. Thereafter cool to room temperature, wash to neutral with water, and then vacuum dry to obtain a solid-phase synthesis carrier with an amino content of 554 µmmol/g and an average pore diameter of 54nm measured by mercury intrusion method.

### Example 2

2L of purified water, 20g of polyvinyl alcohol and 30g of sodium chloride are added to a 3L reactor equipped with a condenser, an agitator and a thermometer, and dissolved to obtain an aqueous phase. 77g of methyl styrene, 28g of di (p-vinylphenyl) methane, 14g of p-chloromethyl styrene, 21g of 1,4-dicyano-2-butene, 15g of sorbitol monooleate, 60g of isooctanol, 30g of dibutyl phthalate and 1g of benzoyl peroxide are weighed out and mixed well to obtain an oil phase. The oil phase is added to the reactor, stirred and heated to 70 °C to carry out polymerization for 8h. Wash with hot water after the polymerization is completed. The pore-forming agents are removed by ethanol reflux extraction. Resin with a particle size of 50-100 µm is screened out and collected, and thereafter vacuum dried to obtain a polymer porous resin with a chlorine content of 646 µmmol/g.

50g of the polymer porous resin and 500ml of N,N-dimethylformamide are added to a 1L reactor equipped with a condenser, an agitator and a thermometer, and stirred. Then 35g potassium phthalimide is added and the temperature is raised to 95 °C to carry out reaction for 16 hours. Cool to room temperature after reaction is completed, wash twice with N,N-dimethylformamide, wash to neutral with purified water, and wash three times with absolute ethanol, and then filter and dry the resin. 200g absolute ethanol and 50g hydrazine hydrate are added to the reactor and heated to a temperature of 75 °C to carry out reaction for 16 hours. Thereafter wash three times with ethanol/purified water solution with a volume ratio of 50:50, wash with purified water to neutral, wash three times with absolute ethanol, and filter and dry the resin. 200g absolute ethanol and 50g concentrated hydrochloric acid are added to the reactor and heated to a temperature of 60 °C to carry out reaction for 6h. Thereafter cool to room temperature, wash to neutral with water, and then vacuum dry to obtain a solid-phase synthesis carrier with an amino content of 635 µmmol/g and an average pore diameter of 145nm measured by mercury intrusion method.

### Example 3

2L of purified water, 20g of polyvinyl alcohol and 30g of sodium chloride are added to a 3L reactor equipped with a condenser, an agitator and a thermometer, and dissolved to obtain an aqueous phase. 123g of ethyl styrene, 8g of 1,3-di (p-vinylphenyl) propane, 8.8g of 4-(4-bromobutyl) styrene, 0.2g of acrylonitrile, 3g of polyoxyethylene sorbitol hexastearate, 18g of isododecane, 5g of dibutyl phthalate and 2g of benzoyl peroxide are weighed out and mixed well to obtain an oil phase. The oil phase is added to the reactor, stirred and heated to 65 °C to carry out polymerization for 10h. Wash with hot water after the polymerization is completed. The pore-forming agents are removed by ethanol reflux extraction. Resin with a particle size of 50-100 µm is screened out and collected, and thereafter vacuum dried to obtain a polymer porous resin with a bromine content of 257 µmmol/g.

50g of the polymer porous resin and 600ml of N,N-dimethylformamide are added to a 1L reactor equipped with a condenser, an agitator and a thermometer, and stirred. Then 5.4g of 4-hydroxybenzoic acid, 5.4g of anhydrous potassium carbonate and 0.3g of potassium iodide are added and heated to 75 °C to carry out reaction for 6 hours. After reaction is completed, cool to room temperature, wash to neutral with water, and then vacuum dry to obtain a solid-phase synthesis carrier with a hydroxyl content of 250 µmmol/g and an average pore diameter of 23nm measured by mercury intrusion method.

### Example 4

2L of purified water, 20g of polyvinyl alcohol and 30g of sodium chloride are added to a 3L reactor equipped with a condenser, an agitator and a thermometer, and dissolved to obtain an aqueous phase. 112g of styrene, 9g of 1,4-di(4'-vinyl phenoxy) butane, 9g of p-chloromethyl styrene, 10g of acrylamide, 1g of ethylene glycol fatty acid ester, 30g of toluene, 60g of dibutyl phthalate and 4.5g of benzoyl peroxide are weighed out and mixed well to obtain an oil phase. The oil phase is added to the reactor, stirred and heated to 60 °C to carry out polymerization for 7h. Wash with hot water after the polymerization is completed. The pore-forming agents are removed by ethanol reflux extraction. Resin with a particle size of 50-100 µm is screened out and collected, and thereafter vacuum dried to obtain a polymer porous resin with a chlorine content of 418 µmmol/g.

50g of the polymer porous resin and 300ml of absolute alcohol are added to a 1L reactor equipped with a condenser, an agitator and a thermometer, and stirred. Then 30g of sodium hydroxide is weighted in a beaker and dissolved with 300ml of deionized water and then added to the reactor slowly. Heat to a temperature of 65 °C to carry out reaction for 6h. Thereafter cool to room temperature, wash to neutral with water, and then vacuum dry to obtain a solid-phase synthesis carrier with a hydroxyl content of 410 µmmol/g and an average pore diameter of 38nm measured by mercury intrusion method.

### Example 5

2L of purified water, 20g of polyvinyl alcohol and 30g of sodium chloride are added to a 3L reactor equipped with a condenser, an agitator and a thermometer, and dissolved to obtain an aqueous phase. 83g of styrene, 23g of 1,2-di (p-vinylphenyl) ethane, 18g of N-(4-vinyl-phenyl)-acetamide, 14g of fumaronitrile, 2g of sorbitan sesquioleate, 10g of dodecanol, 10g of cyclohexane and 2g of benzoyl peroxide are weighed out and mixed well to obtain an oil phase. The oil phase is added to the reactor, stirred and heated to 55 °C to carry out polymerization for 10h. Wash with hot water after the polymerization is completed. The pore-forming agents are removed by ethanol reflux extraction. Resin with a particle size of 50-100 µm is screened out and collected, and thereafter vacuum dried to obtain a polymer porous resin.

50g of the polymer porous resin and 300ml of absolute alcohol are added to a 1L reactor equipped with a condenser, an agitator and a thermometer, and stirred. Then 30g of sodium hydroxide is weighted in a beaker and dissolved with 300ml deionized water and then added to the reactor slowly. Heat to a temperature of 65 °C to carry out reaction for 6h. Thereafter cool to room temperature, wash to neutral with water, and then vacuum dry to obtain a solid-phase synthesis carrier with an amino content of 822 µmmol/g and an average pore diameter of 34nm measured by mercury intrusion method.

### Example 6

2L of purified water, 20g of polyvinyl alcohol and 30g of sodium chloride are added to a 3L reactor equipped with a condenser, an agitator and a thermometer, and dissolved to obtain an aqueous phase. 107.7g of styrene, 12.8g of 1,2-di (p-vinylphenyl) ethane, 12.5g of 4-acetoxystyrene, 7g of fumaronitrile, 10g of sorbitan trioleate, 42g of isooctanol, 21g of isododecane and 2.5g of benzoyl peroxide are weighed out and mixed well to obtain an oil phase. The oil phase is added to the reactor, stirred and heated to 78 °C to carry out polymerization for 6h. Wash the resin with hot water after the polymerization is completed. The pore-forming agents are removed by ethanol reflux extraction. Resin with a particle size of 50-100 µm is screened out and collected, and thereafter vacuum dried to obtain a polymer porous resin.

50g of the polymer porous resin and 300ml of acetonitrile are added to a 1L reactor equipped with a condenser, an agitator and a thermometer, and stirred. Then 7.5ml hydrazine hydrate is added slowly and react for 3h at room temperature. Thereafter wash to neutral with water, and then vacuum dry to obtain a solid-phase synthesis carrier with a hydroxyl content of 538 µmmol/g and an average pore diameter of 58nm measured by mercury intrusion method.

### Example 7

2L of purified water, 20g of polyvinyl alcohol and 30g of sodium chloride are added to a 3L reactor equipped with a condenser, an agitator and a thermometer, and dissolved to obtain an aqueous phase. 128g of styrene, 8g of 1,3-di (p-vinylphenyl) propane, 3g of 4-acetoxystyrene, 1g of fumaronitrile, 0.4g of polyoxyethylene sorbitol hexastearate, 35g of dibutyl phthalate, 12g toluene and 3.5g of benzoyl peroxide are weighed out and mixed well to obtain an oil phase. The oil phase is added to the reactor, stirred and heated to 70 °C to carry out polymerization for 6h. Wash with hot water after the polymerization is completed. The pore-forming agents are removed by ethanol reflux extraction. Resin with a particle size of 50-100 µm is screened out and collected, and thereafter vacuum dried to obtain a polymer porous resin.

50g of the polymer porous resin and 300ml of acetonitrile are added to a 1L reactor equipped with a condenser, an agitator and a thermometer, and stirred. Then 7.5ml hydrazine hydrate is added slowly and react for 3h at room temperature. Thereafter wash to neutral with water, and then vacuum dry to obtain a solid-phase synthesis carrier with a hydroxyl content of 125 µmmol/g and an average pore diameter of 46nm measured by mercury intrusion method.

### Example 8

2L of purified water, 20g of polyvinyl alcohol and 30g of sodium chloride are added to a 3L reactor equipped with a condenser, an agitator and a thermometer, and dissolved to obtain an aqueous phase. 83g of styrene, 17g of 1,4-di(4'-vinylphenoxy) butane, 27g of benzoyloxy styrene, 13g of fumaronitrile, 103g of isooctanol, 51g of isododecane and 3g of benzoyl peroxide are weighed out and mixed well to obtain an oil phase. The oil phase is added to the reactor, stirred and heated to 80 °C to carry out polymerization for 6h. Wash the resin with hot water after the polymerization is completed. The pore-forming agents are removed by ethanol reflux extraction. Resin with a particle size of 50-100 µm is screened out and collected, and thereafter vacuum dried to obtain a polymer porous resin.

50g of the polymer porous resin and 300ml of acetonitrile are added to a 1L reactor equipped with a condenser, an agitator and a thermometer, and stirred. Then 7.5ml hydrazine hydrate is added slowly and react for 3h at room temperature. Thereafter wash to neutral with water, and then vacuum dry to obtain a solid-phase synthesis carrier with a hydroxyl content of 843 µmmol/g and an average pore diameter of 42nm measured by mercury intrusion method.

### Example 9

2L of purified water, 20g of polyvinyl alcohol and 30g of sodium chloride are added to a 3L reactor equipped with a condenser, an agitator and a thermometer, and dissolved to obtain an aqueous phase. 85g of styrene, 24g of 1,2-di (p-vinylphenyl) ethane, 18g of methyl 4-vinylbenzoate, 13g of fumaronitrile, 4g of propylene glycol laurate, 40g of dibutyl phthalate, 40g of isododecane and 2.5g of benzoyl peroxide are weighed out and mixed well to obtain an oil phase. The oil phase is added to the reactor, stirred and heated to 70 °C to carry out polymerization for 6h. Wash the resin with hot water after the polymerization is completed. The pore-forming agents are removed by ethanol reflux extraction. Resin with a particle size of 50-100 µm is screened out and collected, and thereafter vacuum dried to obtain a polymer porous resin.

50g of the polymer porous resin and 300ml of acetonitrile are added to a 1L reactor equipped with a condenser, an agitator and a thermometer, and stirred. Then 8.8g of hydroquinone, 23g of HBTU and 13ml of DIEA are added and react for 2h at room temperature. Thereafter wash to neutral with water, and then vacuum dry to obtain a solid-phase synthesis carrier with a hydroxyl content of 724 µmmol/g and an average pore diameter of 124nm measured by mercury intrusion method.

### Comparative Example 1

2L purified water, 20g of polyvinyl alcohol and 60g of sodium chloride are added to a 3L reactor equipped with a condenser, an agitator and a thermometer, and dissolved to obtain an aqueous phase. 111g of styrene, 11g of divinylbenzene (80% by weight), 13g of 4-acetoxystyrene, 5g of fumaronitrile, 8g of sorbitol monooleate, 40g of isooctanol, 20g of isododecane and 2.5g of benzoyl peroxide are weighed out and mixed well to obtain an oil phase. The oil phase is added to the reactor, stirred and heated to 78 °C to carry out polymerization for 6h. Wash the resin with hot water after the polymerization is completed. The pore-forming agents are removed by ethanol reflux extraction. Resin with a particle size of 50-100 µm is screened out and collected, and thereafter vacuum dried to obtain a polymer porous resin.

50g of the polymer porous resin and 300ml of acetonitrile are added to a 1L reactor equipped with a condenser, an agitator and a thermometer, and stirred. Then 7.5ml hydrazine hydrate is added and react for 3h at room temperature. Thereafter wash to neutral with water, and then vacuum dry to obtain a solid-phase synthesis carrier with a hydroxyl content of 550 µmmol/g and an average pore diameter of 64nm measured by mercury intrusion method.

### Example 10

Swelling properties of the solid-phase synthesis carriers prepared in Examples 1-9 in acetonitrile and toluene are measured, respectively. The method is as follows: About 1.5g of the sample are weighed and put into a stoppered measuring cylinder. Toluene or acetonitrile are added to the corresponding scale. Then the resins and solvent are stirred with a glass rod to make it fully swell and tighten the stopper. 2-3 hours later, the resins are stirred slowly with a glass rod to remove the air bubbles and make the resin uniformly dispersed without caking. The stirring rod is removed and the measuring cylinder is placed on a table with a rubber mat and vibrates to make the resin packed compactly. After standing for 24 hours, the volume is recorded and swelling degree is calculated.

The results are shown in Table 1:

| Table 1. Swelling degree tests of resin | | | | | | | | | |
|---|---|---|---|---|---|---|---|---|---|
| | Example 1 | Example 2 | Example 3 | Example 4 | Example 5 | Example 6 | Example 7 | Example 8 | Example 9 |
| Swelling degree in acetonitrile (ml/g) | 3.8 | 3.7 | 3.4 | 3.6 | 3.6 | 3.8 | 3.7 | 3.8 | 3.8 |
| Swelling degree in toluene (ml/g) | 5.3 | 6.0 | 4.8 | 5.0 | 5.2 | 5.4 | 5.3 | 5.2 | 5.9 |

### Example 11

The solid-phase synthesis carriers prepared in the Examples 1, 2, 6 and 9 and Comparative Example 1 and NittoPhase HL solid-phase synthesis carriers are selected to synthesize oligonucleotide fragments and the performances of the resins are evaluated. To better show the advantages of the present invention, the loading amount (g) of carriers = synthesis column volume (ml) / swelling degree of carrier in toluene (ml/g), and the wash volume during the synthesis of oligonucleotides is limited to the volume of one synthesis column.

10g of the solid-phase synthesis carrier is weighed in a reactor and swollen in 50ml of acetonitrile for 10min, and then 3.0g of DMT-dT-3'- succinic acid, 1.5g of HBTU and 1.3ml of DIEA are added thereto to carry out reaction at room temperature for 12h. After the reaction is completed, wash 5 times with acetonitrile. Then Cap A (consisting of 20ml of acetonitrile, 7.5ml of pyridine and 5.0ml of N-methylimidazole) and Cap B (consisting of 10ml of acetonitrile and 4ml of acetic anhydride) are added to carry out reaction at room temperature for 30min. After the reaction is completed, the resin is washed 5 times with acetonitrile and dried under vacuum to obtain the carrier loaded with DMT-dT. The loaded DMT groups are removed using p-toluenesulfonic acid/acetonitrile solution. Contents of the DMT group loaded in the carrier are determined by spectrophotometry at 412 nm, and the results are shown in Table 2.

The carriers loaded with DMT-dT are weighed and filled in a synthesis column (32 ml). The synthesis column is mounted on AKTA OligoPilot 100 to synthesize an oligonucleotide with 20 bases in length having a sequence of d[ACGTACGTACGTACGTACGT]. The synthesis process is as follows: 1. Swell the resin in dichloromethane; 2. Remove DMT groups with 10% DCA/DCM; 3. Wash with anhydrous acetonitrile; 4. Add phosphoramidite monomer and activating reagent to carry out condensation; 5. Wash with anhydrous acetonitrile; 6. Add oxidizing agent to carry out oxidation; 7. Wash with anhydrous acetonitrile; 8. Add capping reagent to carry out end capping; 9. Wash with anhydrous acetonitrile; and 10. Repeat the step 2 to start the next cycle.

After the synthesis is completed, the carriers are taken out for drying. Then they are put into a glass bottle, and an appropriate amount of concentrated ammonia water is added thereto to carry out reaction at 55°C for 16h, so that the oligonucleotides are cleaved from the carriers while the protecting groups on the bases are removed. The carrier and oligonucleotide are separated via filtration, and the filtrate is dried to obtain a crude powder of oligonucleotide. Purity of the oligonucleotide is measured by HPLC and yield of the oligonucleotide is calculated. The results are shown in Table 2.

| Table 2. Test of resin properties | | | | | | |
|---|---|---|---|---|---|---|
| | Example 1 | Example 2 | Example 6 | Example 9 | Comparative Example 1 | NittoPhase HL |
| Content of DMT group (µmmol/g) | 324 | 252 | 329 | 346 | 318 | 321* |
| Swelling degree in toluene (ml/g) | 5.3 | 6.0 | 5.4 | 5.9 | 5.5 | 5.8 |
| Loading amount of the carrier (g) | 6.0 | 5.3 | 5.9 | 5.4 | 5.8 | 5.5 |
| HPLC Purity (%) | 88.5 | 89.7 | 89.3 | 86.3 | 85.4 | 79.5 |
| Yield (%) | 67.9 | 67.3 | 68.6 | 65.5 | 65.1 | 60.3 |
| Note: *NittoPhase HL loading UnyLinker. | | | | | | |

As can be seen from Table 2, the use of the oligonucleotide solid-phase synthesis carrier of the present invention can improve the yield and purity of oligonucleotides and thus help reduce the production cost of oligonucleotides.

The above examples and technical solutions are only for preferred embodiments of the present invention. It should be pointed out that for those skilled in the art, any changes or alterations derived from the spirit of the present invention are still within the protection scope of the present invention.

## Claims

1. A solid-phase synthesis carrier, **characterized in that** the carrier has a polymer skeleton with functional groups which is represented by the following formula: 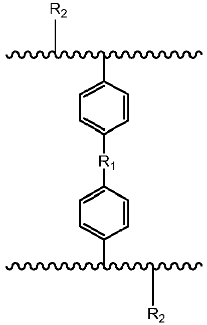 wherein, R₁= -(CH₂)ₙ-, n is an integer of 0-3, or R₁= -O-(CH₂)ₘ-O-, m is an integer of 1-4; and R₂= -OH or -NH₂.

2. The solid-phase synthesis carrier according to claim 1, **characterized in that** the carrier has a content of hydroxyl group or amino group of 100-1000 µmmol/g.

3. The solid-phase synthesis carrier according to claim 2, **characterized in that** the carrier has a content of hydroxyl group or amino group of 400-700 µmmol/g.

4. The solid-phase synthesis carrier according to claim 1, **characterized in that** the carrier has a particle size in a range of 35-200 µm.

5. The solid-phase synthesis carrier according to claim 4, **characterized in that** the carrier has a particle size in a range of 50-100 µm.

6. The solid-phase synthesis carrier according to claim 1, **characterized in that** the carrier has an average pore diameter of 10-200nm.

7. The solid-phase synthesis carrier according to claim 6, **characterized in that** the carrier has an average pore diameter of 40-100nm.

8. A method for preparing the solid-phase synthesis carrier according to claim 1, **characterized by** comprising the following steps of:
A. preparing an aqueous phase and an oil phase, respectively; the aqueous phase comprising water, a dispersant and an inorganic salt; the oil phase comprising: a cross-linking monomer, a monovinyl compound, a functional monomer, a modified monomer, a pore-forming agent and an initiator, wherein the cross-linking monomer, the monovinyl compound, the functional monomer and the modified monomer are monomers capable of polymerization; and
B. adding the oil phase to the aqueous phase, stirring and heating to carry out reaction, and removing the pore-forming agent after the reaction is completed, obtaining a porous polymer resin.

9. The method according to claim 8, **characterized in that** the porous polymer resin is capable of undergoing a further reaction to obtain a solid-phase synthesis carrier containing a hydroxyl group or an amino group as functional groups.

10. The method according to claim 8, **characterized in that** the cross-linking monomer is a diene cross-linking agent which has two vinyl groups not on the same benzene ring.

11. The method according to claim 10, **characterized in that** the cross-linking monomer is selected from the group consisting of 4,4'-divinylbiphenyl, di(4-vinylphenyl) methane, 1,2-di(4-vinylphenyl) ethane, 1,3-di(4-vinylphenyl) propane, di(4'-vinylphenoxy) methane, 1,2-di(4'-vinylphenoxy) ethane, 1,3-di(4'-vinylphenoxy) propane, 1,4-di(4'-vinylphenoxy) butane and any combination thereof.

12. The method according to claim 8, **characterized in that** the monovinyl compound is an aromatic monovinyl compound.

13. The method according to claim 12, **characterized in that** the monovinyl compound is styrene, unsubstituted or substituted with C1-C5 alkyl or alkoxy on its benzene ring.

14. The method according to claim 8, **characterized in that** the functional monomer has a double bond capable of free radical polymerization, and also has a hydroxyl group, an amino group, a halogenated group or other group capable of converting into a hydroxyl group and an amino group via reaction.

15. The method according to claim 14, **characterized in that** the functional monomer is selected from the group consisting of hydroxystyrene and derivatives thereof, such as 4-hydroxystyrene; hydroxyalkyl styrene and derivatives thereof, such as 4-hydroxymethyl styrene; acyloxy styrene and derivatives thereof, such as 4-acetoxy styrene and benzoyloxy styrene; amino styrene and derivatives thereof, such as 4-amino styrene; aminoalkyl styrene and derivatives thereof, such as 4-aminomethyl styrene; haloalkyl styrene monomers, such as 4-(4-bromobutyl) styrene and p-chloromethyl styrene; 4-vinylphenyl ester monomers, such as methyl 4-vinylbenzoate, and 4-ethenylbenzeneacetic acid ethyl ester.

16. The method according to claim 8, **characterized in that** the modified monomer has a double bond capable of free radical polymerization and also has cyano group, ester group and amide group.

17. The method according to claim 16, **characterized in that** the modified monomer is selected from the group consisting of acrylonitrile, methacrylonitrile, fumaronitrile, 1,4-dicyano-2-butene, methyl methacrylate and acrylamide.

18. The method according to claim 8, **characterized in that** the initiator is an organic peroxide or an azo compound.

19. The method according to claim 18, **characterized in that** the initiator is selected from the group consisting of benzoyl peroxide, lauroyl peroxide, tert butyl peroxy-2-ethylhexanoate, 2,2'-azobis(2-methylpropionitrile), 2,2'-azobis(2-methylbutyronitrile) and 2,2'-azobis(2,4-dimethyl)valeronitrile.

20. The method according to claim 8, **characterized in that** the pore-forming agent is an organic solvent or a surfactant which is not polymerizable and insoluble or slightly soluble in water.

21. The method according to claim 20, **characterized in that** the pore-forming agent is selected from the group consisting of aromatic hydrocarbon such as benzene, toluene and ethylbenzene; aliphatic hydrocarbons, such as C6-C12 linear or branched alkanes or C6-C12 cycloalkanes, such as hexane, heptane, octane, dodecane, isooctane, isododecane and cyclohexane; halogenated hydrocarbons such as chloroform and chlorobenzene; esters containing 4 or more carbon atoms, such as ethyl acetate, butyl acetate and dibutyl phthalate; alcohols, such as C4-C12 linear or branched alkane alcohol or C4-C12 cycloalkane alcohol, such as hexanol, cyclohexanol, octanol, isooctanol, decanol and dodecanol; oil-soluble surfactants such as sorbitan trioleate, polyoxyethylene sorbitol beeswax derivative, sorbitan tristearate, polyoxyethylene sorbitol hexastearate, ethylene glycol fatty acid ester, propylene glycol fatty acid ester, propylene glycol monostearate, sorbitan sesquioleate, polyoxyethylene sorbitol oleate, monostearin, lanolin hydroxylated, sorbitol monooleate and propylene glycol laurate, and any combination thereof.

22. The method according to claim 8, **characterized in that**,
the dispersant is present in an amount of 0.1-5% by weight in the aqueous phase, and the inorganic salt is present in an amount of 20% by weight or lower in the aqueous phase;
a weight ratio of the oil phase to the aqueous phase is 1:3-1:20;
the monovinyl compound in the oil phase accounts for 45-95% by weight based on a total weight of the monomers;
the cross-linking monomer in the oil phase accounts for 2.9-20% by weight based on a total weight of the monomers;
the functional monomers in the oil phase accounts for 2-20% by weight based on a total weight of the monomers;
the modified monomer in the oil phase accounts for 0.1-15% by weight based on a total weight of the monomers; and
the pore-forming agent in the oil phase accounts for 15-130% by weight based on a total weight of the monomers.

23. The method according to claim 22, **characterized in that**,
the monovinyl compound in the oil phase accounts for 62-86% by weight based on a total weight of the monomers;
the cross-linking monomer in the oil phase accounts for 7-13% by weight based on a total weight of the monomers;
the functional monomers in the oil phase accounts for 5-15% by weight based on a total weight of the monomers;
the modified monomer in the oil phase accounts for 2-10% by weight based on a total weight of the monomers; and
the pore-forming agent in the oil phase accounts for 30-110% by weight based on a total weight of the monomers.

24. The method according to claim 8, **characterized in that** the polymerization is carried out at a temperature of 50-90 °C.

25. The method according to claim 24, **characterized in that** the polymerization is carried out at a temperature of 70-85 °C.

26. The method according to any one of claims 8-25, **characterized by** comprising the following steps of:
adding a certain amount of purified water into a reactor, adding the dispersant in an amount which is 0.1-5% by weight of the aqueous phase and the inorganic salt in an amount which is not more than 20% by weight of the aqueous phase, and dissolving to obtain an aqueous phase;
weighing out the monovinyl compound, cross-linking monomer, functional monomer, modified monomer, pore-forming agent and initiator according to a weight ratio of the oil phase to the aqueous phase being1:3-1:20;, wherein, the monovinyl compound accounts for 45-95% of the total weight of the monomers, the cross-linking monomer accounts for 2.9-20% of the total weight of the monomers, the functional monomer accounts for 2-20% of the total weight of the monomers, and the modified monomer accounts for 0.1-15% of the total weight of the monomers, the pore-forming agent accounts for 15-130% of the total weight of monomers, and mixing well to obtain an oil phase;
adding the oil phase into the reactor, stirring and heating up to 50-90 °C to carry out reaction; removing the pore-forming agent after the reaction is completed, and screening and collecting the resin with appropriate particle size, and vacuum drying to obtain a porous polymer resin; and
carrying out a further reaction with the resin to obtain a solid-phase synthesis carrier having amino group or carboxyl group.

27. The method according to claim 26, **characterized by** comprising the following steps of:
adding a certain amount of purified water into a reactor, adding the dispersant in an amount which is 0.1-5% by weight of the aqueous phase and the inorganic salt in an amount which is not more than 20% by weight of the aqueous phase, and dissolving to obtain an aqueous phase;
weighing out the monovinyl compound, cross-linking monomer, functional monomer, modified monomer, pore-forming agent and initiator according to a weight ratio of the oil phase to the aqueous phase being 1:3-1:20;, wherein, the monovinyl compound accounts for 62-86% of the total weight of the monomers, the cross-linking monomer accounts for 7-13% of the total weight of the monomers, the functional monomer accounts for 5-15% of the total weight of the monomers, and the modified monomer accounts for 2-10% of the total weight of the monomers, the pore-forming agent accounts for 30-110% of the total weight of monomers, and mixing well to obtain an oil phase;
adding the oil phase into the reactor, stirring and heating up to 70-85 °C to carry out reaction; removing the pore-forming agent after the reaction is completed, and screening and collecting the resin with appropriate particle size, and vacuum drying to obtain a porous polymer resin; and
carrying out a further reaction with the resin to obtain a solid-phase synthesis carrier having amino group or carboxyl group.

28. The method according to any one of claims 8-27, **characterized by** comprising the following steps of:
adding 2L of purified water, 20g of polyvinyl alcohol and 30g of sodium chloride into a 3L reactor equipped with a condenser, an agitator and a thermometer, and dissolving to obtain an aqueous phase;
weighing out 108.8g of styrene, 14g of 1,2-di (p-vinylphenyl) ethane, 12.2g of p-chloromethyl styrene, 5g of fumaronitrile, 6g of sorbitol trioleate, 40g of isooctanol, 20g of isododecane and 2.5g of benzoyl peroxide, and mixing well to obtain an oil phase;
adding the oil phase into the reactor, stirring, and heating up to 80 °C to carry out polymerization for 6h; washing with hot water after the polymerization is completed, removing the pore-forming agent by ethanol reflux extraction, screening and collecting the resin a particle size of 50-100 µm and vacuum drying to obtain a polymer porous resin with a chlorine content of 565µmol/g;
adding 50g of the polymer porous resin and 500ml of N,N-dimethylformamide into a 1L reactor equipped with a condenser, an agitator and a thermometer, and stirring; then adding 30g of potassium phthalate and heating up to 95 °C to carry out reaction for 16 hours; cooling to room temperature after the reaction is completed, washing twice with N,N-dimethylformamide, washing to neutral with purified water, washing three times with absolute ethanol, and filtering and drying the resin; adding 200g of absolute ethanol and 50g of hydrazine hydrate into the reactor, heating up to 75 °C and reacting for 16 hours; thereafter washing three times with ethanol/purified water solution with a volume ratio of 50:50, washing to neutral with purified water, washing three times with absolute ethanol, and filtering and drying, adding 200g of absolute ethanol and 50g of concentrated hydrochloric acid to the reactor, heating up to 60 °C and reacting for 6h, thereafter cooling to room temperature, washing to neutral with water, and then vacuum drying to obtain a solid-phase synthesis carrier having an amino content of 554 µmol/g and an average pore diameter of 54nm measured by mercury intrusion method.

29. The method according to any one of claims 8-27, **characterized by** comprising the following steps of:
adding 2L of purified water, 20g of polyvinyl alcohol and 30g of sodium chloride into a 3L reactor equipped with a condenser, an agitator and a thermometer, and dissolving to obtain an aqueous phase;
weighing out 107.7g of styrene, 12.8g of 1,2-di (p-vinylphenyl) ethane, 12.5g of 4-acetoxystyrene, 7g of fumaronitrile, 10g of sorbitol trioleate, 42g of isooctanol, 21g of isododecane and 2.5g of benzoyl peroxide, and mixing well to obtain an oil phase;
adding the oil phase into the reactor, stirring, and heating up to 78°C to carry out polymerization for 6h; washing with hot water after the polymerization is completed, removing the pore-forming agent by ethanol reflux extraction, screening and collecting the resin a particle size of 50-100 µm and vacuum drying to obtain a polymer porous resin;
adding 50g of the polymer porous resin and 300ml of acetonitrile into a 1L reactor equipped with a condenser, an agitator and a thermometer, and stirring; then adding 7.5ml of hydrazine hydrate slowly and reacting for 3h at room temperature, washing to neutral with water, and then vacuum drying to obtain a solid-phase synthesis carrier having an hydroxyl content of 538µmol/g and an average pore diameter of 58nm measured by mercury intrusion method.

30. Use of the solid-phase synthesis carrier of claim 1 prepared via the method according to any one of claims 8-29 in the solid-phase synthesis of oligonucleotides.

31. The solid-phase synthesis carrier according to any one of claims 1-7, **characterized in that** the solid-phase synthesis carrier is a copolymer comprising repeating structural units represented by formula (I), formula (II), formula (III), and formula (IV) in its skeleton: 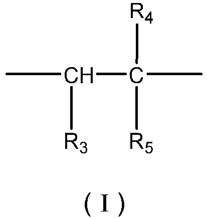
wherein, R₃ is selected from the groups consisting of -H, -CN and -CH₂-CN; R₄ is -H or -CH₃; and R₅ is selected from the groups consisting of -CN, -CH₂-CN, -OOCH₃ and -CONH₂; 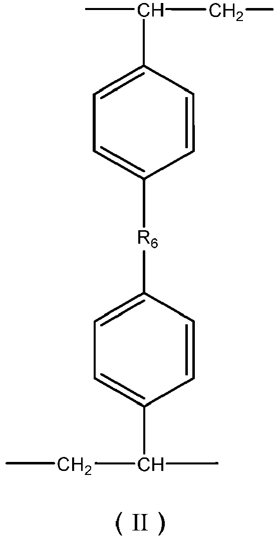
wherein, R₆ is -(CH₂₎ₓ-, x is an integer 0-3, or R6 is -O-(CH₂)_{y}-O -, y is an integer 1-4; 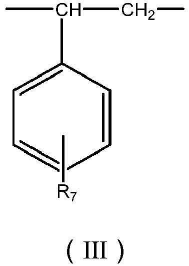
R₇ is selected from the groups consisting of -H, CH₃(CH₂)_{z} - or CH₃(CH₂)_{Z}O-, wherein z is an integer 0-4, (CH₃)₂CH-, (CH₃)₂CH(CH₂)-, (CH₃)₂CH(CH₂)₂-, (CH₃)₃C-, CH₃CH₂CH(CH₃)-, CH₃CH₂C(CH₃)₂-, and CH₃CH₂CH₂CH(CH₃)-; 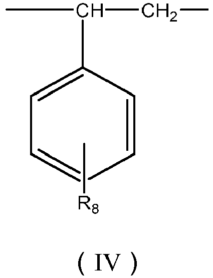
R₈ is selected from the groups consisting of -OH, -CH₂OH, -NH₂, -CH₂NH₂,-CHCOOC-C₆H₄-OH, -CHCOOCCH₂-C₆H₄-OH, -(CH₂)₄OOC-C₆H₄-OH,-(CH₂)₄OOCCH₂-C₆H₄-OH, -(CH₂)₄OOCCH₂-C₆H₄-NH₂, -CH₂COONH-C₆H₄-NH₂,-COO-C₆H₄-OH and -CH₂COO-C₆H₄-OH.
